# EUROPÄISCHE PATENTSCHRIFT

(11) **EP 3 553 139 B1**
(45) Veröffentlichungstag und Bekanntmachung des Hinweises auf die Patenterteilung: **29.04.2020**
(21) Anmeldenummer: 18167327.8
(22) Anmeldetag: 13.04.2018
(51) Int. Cl.: C09D 11/17, A61K 8/00

(54) **WEISSLICHE SCHREIB- UND MARKIERFLÜSSIGKEIT FÜR KAPILLARSYSTEME**
WHITE WRITING AND MARKING LIQUID FOR CAPILLARY SYSTEMS
LIQUIDE D'ÉCRITURE ET DE MARQUAGE BLANCHÂTRE POUR SYSTÈMES CAPILLAIRES

(43) Veröffentlichungstag der Anmeldung: 16.10.2019
(73) Patentinhaber: Faber- Castell AG, 90546 Stein (DE)
(72) Erfinder: Appel, Tatiana, 90522 Oberasbach (DE); Lugert, Gerhard, 90431 Nürnberg (DE); Seidl, Kurt, 4072 Alkoven (AT)
(74) Vertreter: Schlögl, Markus

(56) Entgegenhaltungen:
- EP-A1- 2 514 792
- EP-A1- 2 891 691
- WO-A1-2015/142335
- US-A1- 2007 060 670
- US-A1- 2015 184 010

## Beschreibung

Die Erfindung betrifft eine weißliche Schreib- und/oder Markierflüssigkeit, insbesondere weiße Schreib- und/oder Markierflüssigkeit, für Schreibgeräte, insbesondere für Kapillarschreibgeräte, oder Kosmetikanwendungen und ein Schreibgerät, in welchem eine solche Schreib- und/oder Markierflüssigkeit verwendet wird.

Farbige Markierflüssigkeiten für sogenannte Marker auf Basis organischer Lösemittel wie Alkohole, Ester, Ketone oder aromatischer Lösemittel wie Toluol oder Xylol sind seit langem bekannt. Als Farbmittel werden zumeist Pigmente, kationische Farbstoffe, sogenannte Fettfarbstoffe oder Metallkomplexfarbstoffe zusammen mit einem oder mehreren Netzmitteln und Harzen verwendet. Weiße oder helle pastellfarbige Schreib- und/oder Markierflüssigkeit mit hohem Weißgrad und einem hohen Deckvermögen können lediglich auf Basis von Pigmenten erzielt werden. Weiße Farbpigmente sind z.B. Kaolin, Kreide oder Titandioxid, wobei Schreib- und Markierflüssigkeiten basierend auf Titandioxid ein höheres Deckungsvermögen und einen höheren Weißgrad aufweisen. Die bekannten Schreib- und Markierflüssigkeiten mit Titandioxidpigmenten haben allerdings eine sehr hohe Dichte und neigen dazu, stark zu sedimentieren. Der Sedimentation kann durch eine Erhöhung der Viskosität einer solchen Flüssigkeit auf Werte deutlich größer 20cP (Methode Brookfield, 20 °C) entgegengewirkt werden. Allerdings können sehr hochviskose Flüssigkeiten nur noch als Farbe aufgebracht werden.

Schreibgeräte, in welchen solche Schreib- und/oder Markierflüssigkeiten eingesetzt werden, umfassen einen Schaft, typischerweise ein Kunststoff- oder Metallgehäuse, auf welchen im Nichtgebrauchszustand eine Verschlusskappe aufgesteckt wird. Innerhalb des Schaftes ist ein Speicher für die Schreib- und/oder Markierflüssigkeit aus einem kapillaren Material, beispielsweise aus Polyester- oder Polyolefinfasern angeordnet, der mit einem Auftragselement aus einem ebenfalls kapillaren Material, z.B. einer Faser- oder Sinterspitze fluidisch verbunden ist, sodass die Schreib- und/oder Markierflüssigkeit aufgrund von Adhäsionskräften bzw. aufgrund des Kapillareffektes aus dem Speicher an die Spitze des Auftragselementes transportiert werden kann.

Bei einer anderen Variante eines Schreibgerätes wird ein sogenanntes Ventilsystem eingesetzt, wobei die Schreib- und/oder Markierflüssigkeit als frei fließende Flüssigkeit in ein Reservoir innerhalb des Schaftes gefüllt ist und eine Flüssigkeitsabgabe über eine Schreib- oder Markierspitze, vorzugsweise aus Polyester- oder Acrylfasern oder auch aus gesinterten Polyolefinen stattfindet. Zur geregelten Flüssigkeitsabgabe ist zwischen der Spitze und dem Reservoir ein Ventil vorgesehen.

Bei letzterem System werden insbesondere bei hochviskosen, pigmentierten Flüssigkeiten häufig eine oder mehrere Mischkugeln in der Flüssigkeit eingesetzt, um vor der Applikation der Flüssigkeit durch Schütteln des Schreibgerätes eine Homogenisierung der Flüssigkeit herbeizuführen. Zum Auftragen der Flüssigkeit ist darüber hinaus auch ein Pumpen notwendig. Dieses notwendige Schütteln und Pumpen wird von den Nutzern als Nachteil empfunden. Das Pumpsystem erlaubt weder die Verwendung von sehr feinen Spitzen noch ermöglicht es ein sehr präzises Applikationsverhalten.

Bei Kapillarsystemen ist der Einsatz solcher Mischkugeln nicht möglich. Die in Kapillarsystemen eingesetzten Flüssigkeiten müssen zur Sicherstellung einer ausreichenden Applikation außerdem sehr geringe Viskositäten von kleiner 50 mPa s, bevorzugt jedoch kleiner 20 mPa s (Brookfield, 20°C, Kegel-Platte CPE-40) besitzen.

Sogenannte Industriemarker enthalten zumeist höherviskose weiße oder farbige Schreib- und/oder Markierflüssigkeiten und können aufgrund der hohen Viskosität (größer 50 mPa s, 20 °C Brookfield, Kegel-Platte CPE-40) nicht durch herkömmliche Kapillarsysteme, sondern nur über Ventilsysteme appliziert werden.

Es ist daher Aufgabe der Erfindung, eine weißliche Schreib- und/oder Markierflüssigkeit, insbesondere eine weiße Schreib- und/oder Markierflüssigkeit, für ein Schreibgerät, insbesondere ein Kapillarschreibgerät, oder Kosmetikanwendungen vorzuschlagen, welche hinsichtlich der geschilderten Nachteile und Probleme verbessert ist. Ferner ist es Aufgabe der Erfindung, ein Schreibgerät, insbesondere ein Kapillarschreibgerät anzugeben, in welchem die Schreib- und/oder Markierflüssigkeit zum Einsatz kommt.

Die erfindungsgemäße weißliche Schreib- und/oder Markierflüssigkeit, insbesondere weiße Schreib- und/oder Markierflüssigkeit, für Schreibgeräte, insbesondere für Kapillarschreibgeräte, oder Kosmetikanwendungen weist eine Viskosität von weniger als 30 mPa s (Brookfield, 20 °C, Kegel-Platte CPE-40) auf. Die Schreib- und/oder Markierflüssigkeit enthält eine wässrige Titandioxid-Dispersion, 5 bis 15 Gew.% einer Dispersion eines Hartharzes und ein Konservierungsmittel.

Unter weißliche Schreib- und/oder Markierflüssigkeit wird eine Schreib- und/oder Markierflüssigkeit verstanden, die einen hohen Weißanteil aufweist, insbesondere also weiß ist oder eine leichte pastellfarbene Abtönung aufweist.

Erstaunlicherweise hat sich herausgestellt, dass eine solche Schreib- und/oder Markierflüssigkeit so stabilisiert ist, dass sie über mehrere Monate hinweg und in Kapillarsystemen verwendet werden kann, ohne dass störende Ablagerungen im Kapillarspeicher der Spitze auftreten. Eine erfindungsgemäße Titandioxid-Dispersion (Titandioxid Colour Index Pigment White 6 (77891), CAS-Nr. 13463-67-7) als Pigmentpräparation, weiterhin auch als Titanweißpaste bezeichnet, weist insbesondere zwischen 70 bis 75 Gew. % Feststoffgehalt auf, wobei das Pigment Rutil, d.h. tetragonales TiO₂, ist. Die Titanweißpaste hat zweckmäßigerweise eine Dichte nach ISO 787/10 von 2,2 ± 0,1 g/cm³, eine Viskosität von 400 mPa s und einen pH-Wert zwischen 7,5 bis 8,5. Die Titanweißpaste hat weiterhin einen Pigmentpartikeldurchmesser D90 von 0,3 µm bei Messung des Durchmessers beispielsweise mit der Methode CILAS 920 nass und einen mittleren Pigmentpartikeldurchmesser D50 von 0,25 µm.

In der Schreib- und/oder Markierflüssigkeit sind insbesondere 45 bis 70 Gew.%, bevorzugt 50 bis 65 Gew. %, bevorzugt 55 bis 60 Gew. %, von der Titanweißpaste enthalten.

In bevorzugter Ausgestaltung ist das Hartharz ein Polyurethanpolyolharz, und die Dispersion eines Hartharzes eine anionische Dispersion eines Polyurethanpolyolharzes.

Eine anionische Dispersion eines Polyurethanpolyolharzes weist insbesondere die folgenden Eigenschaften auf:

| | |
|---|---|
| Viskosität bei 23 °C (Lieferform) | ca. 400 mPa s |
| pH-Wert (Lieferform) | ca. 9 |
| Lösemittel | Wasser |

Eine solche anionische Dispersion eines Polyurethanpolyolharzes ist beispielsweise unter dem Handelsnamen TEGO® VariPlus DS 50¹⁾ erhältlich.

Zweckmäßigerweise werden als Konservierungsmittel eines oder mehrere aus Formaldehydabspalter, Phenoxyethanol, Parabene und Isothiazolinone verwendet.

Ein geeignetes Konservierungsmittel, welches einen Formaldehyabspalter darstellt, ist beispielsweise unter dem Handelsnamen ACTICIDE SR 7034 ³⁾ erhältlich. Das bekannte Konservierungsmittel ist ein Biozid auf Basis von 1,6-Dihydroxi-2,5-dioxahexan und Tetramethylolacetylendiharnstoff, wobei 1,6-Dihydroxi-2,5-dioxahexan zu Formaldehyd und Ethylenglycol unter Umweltbedingungen hydrolisiert.

Ein weiteres geeignetes Konservierungsmittel ist beispielsweise unter dem Handelsnamen ACTICIDE LT2 ⁴⁾ erhältlich. Das Konservierungsmittel ist ein Isothiazolinon-basiertes Biozid, welches 2-Brom-2-nitropropan-1,3-diol und 2-Octyl-2H-isothiazol-3-on enthält.

In Ausgestaltung enthält die Schreib- und/oder Markierflüssigkeit weiterhin 1,0 bis 3,0 Gew.% eines Netzmittels, insbesondere eines Silikonnetzmittels. Zweckmäßigerweise ist das Silikonnetzmittel ein Polyethylen-modifiziertes Polysiloxan. Eine solches Polyethylen-modifiziertes Polysiloxan ist zum Beispiel unter dem Handelsnamen ABIL B 8851 ²⁾ erhältlich. Polyethylen-modifiziertes Polysiloxan ist in Wasser löslich und weist eine dynamische Viskosität von 330 bis 570 mPa s bei 25 °C im Lieferzustand auf.

In Ausgestaltung umfasst die Schreib- und/oder Markierflüssigkeit Feuchthaltemittel, insbesondere Glycerin und/oder Glykole oder Polyalkohole. Als Glykole können beispielsweise Butylenglykol oder Propylenglykol verwendet werden. Zweckmäßigerweise werden bis zu 15 Gew.%, insbesondere 3 bis 10 Gew.%, insbesondere 5 bis 8 Gew.%, Feuchthaltemittel zugesetzt.

In Ausgestaltung umfasst die Schreib- und/oder Markierflüssigkeit weitere Additive ausgewählt aus Antiblockingadditiven, insbesondere eine kolloidale Dispersion aus Siliziumdioxidpartikeln, und/oder Polyether-modifizierten Polydimethylsiloxanen. Erfindungsgemäße Antiblockingadditive werden beispielsweise unter den Handelsnamen Borchi Coll 10 ⁵⁾, Borchi Coll 20 ⁶⁾, Borchi Coll 20M ⁷⁾, Borchi Coll 30 ⁸⁾ und ein Polyether-modifizierte Polydimethylsiloxane unter BYK 3455 ⁹⁾ vertrieben.

Die Antiblockieradditive haben die folgenden Eigenschaften

| | |
|---|---|
| Viskosität bei 23 °C (Lieferform) | 3 - 7 mPa s, insbesondere 5 mPa s |
| pH-Wert (Lieferform) | ca. 8,5 - 10,5, insbesondere 8,5 - 9,5 |

Zweckmäßigerweise werden die weiteren Additive in einer Gesamtmenge von bis zu 10 Gew.% zugesetzt.

In Ausgestaltung kann die Schreib- und/oder Markierflüssigkeit blaue Farbstoffe oder Pigmente in einer Konzentration von maximal 0,2 Gew.% enthalten. Die Zugabe einer geringen Menge blauen Farbstoffs oder blauer Pigmente, z.B. von Patentblau oder Ultramarin erhöht die Farbbrillanz.

In einer alternativen Ausgestaltung kann die Schreib- und/oder Markierflüssigkeit Farbstoffe oder Pigmente in einer Konzentration von maximal 3,0 Gew.% enthalten. Somit können Schreib- und/oder Markierflüssigkeit mit einer farblichen Tönung erhalten werden, die z.B. Pastellgelb oder Pastellrot sind.

Das erfindungsgemäße Schreibgerät umfasst eine Schreibspitze aus einem kapillaren Material und einen eine erfindungsgemäße Schreib- und/oder Markierflüssigkeit enthaltenden Speicher aus einem kapillaren Material.

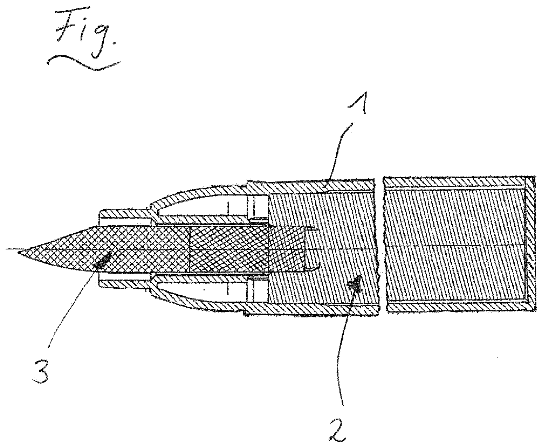

Die Erfindung wird nun nachfolgend anhand von Ausführungsbeispielen und einer Zeichnung (Fig. 1), die ein kapillares Schreibgerät in Längsschnittdarstellung zeigt, näher erläutert.

Das Schreibgerät, z.B. ein Marker, ist im Wesentlichen stiftförmig und weist einen Schaft 1 auf, in dem ein Speicher 2 aus einem kapillaren Material bzw. ein Faserspeicher geringer Dichte, z.B. ein Polyesterfaserspeicher vorhanden ist. Das vordere Stiftende trägt eine Schreibspitze 3 aus einem kapillaren Material bzw. eine Faserspitze, die mit ihrem hinteren Ende mit dem Faserspeicher 2 in Verbindung steht bzw. in diesen hineinragt.

Die Herstellung der Schreib- und/oder Markierflüssigkeit erfolgt überwiegend in Mischkesseln, in welchen der pigmenthaltigen Dispersion die weiteren Komponenten, beispielsweise Farbmittel oder Additive, zugesetzt werden. Dabei wird ständig mit einem Rührer homogenisiert, um die Komponenten intensiv miteinander zu vermischen und ausreichend gleichmäßig zu verteilen. Anschließend wird die so erhaltene Schreib- und/oder Markierflüssigkeit in den Speicher 2 aus kapillarem Material des oben beschriebenen Schreibgerätes eingefüllt.

Nachfolgend sind sieben Beispielrezepturen für weiße Schreib- und Markierflüssigkeiten aufgeführt. Die Angaben in den Beispielrezepturen sind in Gew. % angegeben.

### Beispiel 1

| | |
|---|---|
| Wasser demineralisiert | 27,40 |
| Silikonnetzmittel | 2,00 |
| Polyether-modifiziertes Polydimethylsiloxan | |
| Formaldehydabspalter | 0,40 |
| Isothiazolinon-basiertes Konservierungsmittel | 0,20 |
| Glycerin | |
| Propylenglykol | |
| Polyurethanpolyolharz | 10,00 |
| Titanweißpaste | 60,00 |
| Antiblockingadditiv | |
| Weiteres Farbmittel | |
| | 100,00 |

Eine Schreib- und/oder Markierflüssigkeit mit einer Zusammensetzung entsprechend Beispiel 1 weist auf Grund des hohen Titanweißpastenanteils eine gute Deckkraft bei guter Langzeitstabilität auf.

### Beispiel 2

| | |
|---|---|
| Wasser demineralisiert | 24,40 |
| Silikonnetzmittel | 2,00 |
| Polyether-modifiziertes Polydimethylsiloxan | |
| Formaldehydabspalter | 0,40 |
| Isothiazolinon-basiertes Konservierungsmittel | 0,20 |
| Glycerin | 5,00 |
| Propylenglykol | |
| Polyurethanpolyolharz | 10,00 |
| Titanweißpaste | 58,00 |
| Antiblockingadditiv | |
| Weiteres Farbmittel | |
| | 100,00 |

Eine Schreib- und/oder Markierflüssigkeit mit einer Zusammensetzung entsprechend Beispiel 2 weist einen hohen Anteil an Feuchthaltemittel auf.

### Beispiel 3

| | |
|---|---|
| Wasser demineralisiert | 23,40 |
| Silikonnetzmittel | 2,00 |
| Polyether-modifiziertes Polydimethylsiloxan | 1,00 |
| Formaldehydabspalter | 0,40 |
| Isothiazolinon-basiertes Konservierungsmittel | 0,20 |
| Glycerin | 5,00 |
| Propylenglykol | |
| Polyurethanpolyolharz | 10,00 |
| Titanweißpaste | 58,00 |
| Antiblockingadditiv | |
| Weiteres Farbmittel | |
| | 100,00 |

Eine Schreib- und/oder Markierflüssigkeit mit einer Zusammensetzung entsprechend Beispiel 3 enthält ein Polyether-modifiziertes Polydimethylsiloxan, welches die Untergrundbenetzung und des Verlaufs verbessert, in dem die Oberflächenspannung reduziert wird. Diese Zusammensetzung erlaubt einen Einsatz auf schwierig zu benetzenden Untergründen.

### Beispiel 4

| | |
|---|---|
| Wasser demineralisiert | 21,40 |
| Silikonnetzmittel | 2,00 |
| Polyether-modifiziertes Polydimethylsiloxan | |
| Formaldehydabspalter | 0,40 |
| Isothiazolinon-basiertes Konservierungsmittel | 0,20 |
| Glycerin | |
| Propylenglykol | |
| Polyurethanpolyolharz | 10,00 |
| Titanweißpaste | 60,00 |
| Antiblockingadditiv | 6,00 |
| Weiteres Farbmittel | |
| | 100,00 |

Eine Schreib- und/oder Markierflüssigkeit mit einer Zusammensetzung entsprechend Beispiel 4 enthält ein Antiblockingadditiv, insbesondere eine nicht sedimentierende, kolloidale Dispersion aus Siliziumdioxid Nanopartikeln. Dieses Additiv beeinflusst die Trocknung und verbessert die Haftung der Schreib- und/oder Markierflüssigkeit auf Holz-, Kunststoff- und Metalloberflächen und erhöht ihre Deckkraft.

### Beispiel 5

| | |
|---|---|
| Wasser demineralisiert | 28,90 |
| Silikon netzmittel | 1,00 |
| Polyether-modifiziertes Polydimethylsiloxan | 0,50 |
| Formaldehydabspalter | 0,40 |
| Isothiazolinon-basiertes Konservierungsmittel | 0,20 |
| Glycerin | |
| Propylenglykol | 5,00 |
| Polyurethanpolyolharz | 14,00 |
| Titanweißpaste | 50,00 |
| Antiblockingadditiv | |
| Weiteres Farbmittel | |
| | 100,00 |

Eine Schreib- und/oder Markierflüssigkeit mit einer Zusammensetzung entsprechend Beispiel 5 enthält Propylenglykol als alternatives Feuchthaltemittel und einen vergleichsweise geringen Anteil an Titanweißpaste.

### Beispiel 6

| | |
|---|---|
| Wasser demineralisiert | 16,40 |
| Silikonnetzmittel | 3,00 |
| Polyether-modifiziertes Polydimethylsiloxan | |
| Formaldehydabspalter | 0,40 |
| Isothiazolinon-basiertes Konservierungsmittel | 0,20 |
| Glycerin | 7,00 |
| Propylenglykol | |
| Polyurethanpolyolharz | 6,00 |
| Titanweißpaste | 65,00 |
| Antiblockingadditiv | 2,00 |
| Weiteres Farbmittel | |
| | 100,00 |

Eine Schreib- und/oder Markierflüssigkeit mit einer Zusammensetzung entsprechend Beispiel 6 weist einen hohen Anteil an Titanweißpaste auf.

### Beispiel 7

| | |
|---|---|
| Wasser demineralisiert | 27,20 |
| Silikonnetzmittel | 2,00 |
| Polyether-modifiziertes Polydimethylsiloxan | |
| Formaldehydabspalter | 0,40 |
| Isothiazolinon | 0,20 |
| Glycerin | |
| Propylenglykol | |
| Polyurethanpolyolharz | 10,00 |
| Titanweißpaste | 60,00 |
| Antiblockingadditiv | |
| Weiteres Farbmittel | 0,20 |
| | 100,00 |

Eine Schreib- und/oder Markierflüssigkeit mit einer Zusammensetzung entsprechend Beispiel 7 weist ein zusätzliches Farbmittel, hier Ultramarinblau in Höhe von 0,2 Gew.% auf. Das zusätzliche Farbmittel erzeugt ein leicht blaustichiges Weiß, was zu einer erhöhten Brillanz führt.

### Produktbezeichnungen/Hersteller:

1) Evonik Resource Efficiency GmbH, 45127 Essen, Deutschland
2) Evonik Industries AG, 45127 Essen, Deutschland
3), 4) THOR GmbH, 67346 Speyer, Deutschland
5), 6), 7), 8) OMG Borchers GmbH, 40764 Langenfeld, Deutschland
9) BYK-Chemie GmbH, 46462 Wesel, Deutschland

## Patentansprüche

1. Weißliche Schreib- und/oder Markierflüssigkeit, insbesondere weiße Schreib- und/oder Markierflüssigkeit, für Schreibgeräte, insbesondere für Kapillarschreibgeräte, oder Kosmetikanwendungen, die eine Viskosität von weniger als 30 mPa s (Brookfield, 20 °C, Kegel-Platte CPE-40) aufweist, enthaltend 45 bis 70 Gew.% einer wässrigen Titandioxid-Dispersion, 5 bis 15 Gew.% einer anionischen Dispersion eines Hartharzes, 1,0 bis 3,0 Gew.% eines Silikonnetzmittels und ein Konservierungsmittel, wobei der Feststoffgehalt in der Titandioxid-Dispersion 70 bis 75 Gew.% beträgt.

2. Schreib- und/oder Markierflüssigkeit nach Anspruch 1, welche 50 bis 65 Gew. %, bevorzugt 55 bis 60 Gew. % einer wässrigen Titandioxid-Dispersion enthält.

3. Schreib- und/oder Markierflüssigkeit nach Anspruch 1 oder 2, wobei das Hartharz ein Polyurethanpolyolharz ist.

4. Schreib- und/oder Markierflüssigkeit nach einem der vorhergehenden Ansprüche, wobei als Konservierungsmittel eines oder mehrere aus Formaldehydabspalter, Phenoxyethanol, Parabene und Isothiazolinone verwendet werden.

5. Schreib- und/oder Markierflüssigkeit nach einem der vorhergehenden Ansprüche, wobei das Silikonnetzmittel ein Polyethylen-modifiziertes Polysiloxane ist.

6. Schreib- und/oder Markierflüssigkeit nach einem der vorhergehenden Ansprüche, umfassend Feuchthaltemittel, insbesondere Glycerin und/oder Glykole oder Polyalkohole.

7. Schreib- und/oder Markierflüssigkeit nach Anspruch 6, wobei bis zu 15 Gew.% Feuchthaltemittel zugesetzt werden.

8. Schreib- und/oder Markierflüssigkeit nach einem der vorhergehenden Ansprüche, umfassend weitere Additive ausgewählt aus Antiblockingadditiven, insbesondere eine kolloidale Dispersion aus Siliziumdioxidpartikeln, und/oder Polyether-modifizierten Polydimethylsiloxanen.

9. Schreib- und/oder Markierflüssigkeit nach Anspruch 8, wobei die weiteren Additive in einer Gesamtmenge von bis zu 10 Gew.% zugesetzt werden.

10. Schreib- und/oder Markierflüssigkeit nach einem der vorhergehenden Ansprüche, umfassend blaue Farbstoffe oder Pigmente in einer Konzentration von maximal 0,2 Gew.%.

11. Schreib- und/oder Markierflüssigkeit nach einem der Ansprüche 1 bis 9, umfassend Farbstoffe oder Pigmente in einer Konzentration von maximal 3,0 Gew.%.

12. Schreibgerät mit einer Schreibspitze (3) aus einem kapillaren Material und einem eine Schreib- und/oder Markierflüssigkeit nach einem der vorhergehenden Ansprüche enthaltenden Speicher (2) aus einem kapillaren Material.

## Claims

1. Whitish writing and/or marking fluid, in particular white writing- and/or marking fluid, for writing instruments, in particular for capillary writing instruments, or cosmetic applications, which has a viscosity of less than 30 mPa s (Brookfield, 20°C, cone-plate CPE-40), containing 45 to 70 weight % of an aqueous titanium dioxide dispersion, 5 to 15 weight % of an anionic dispersion of a hard resin, 1.0 to 3.0 weight % of a silicone wetting agent and a preserving agent, wherein the solids content in the titanium dioxide dispersion is 70 to 75 weight %.

2. Writing and/or marking fluid according to claim 1, which contains 50 to 65 weight %, preferably 55 to 60 weight %, of an aqueous titanium dioxide dispersion.

3. Writing and/or marking fluid according to claim 1 or 2, wherein the hard resin is a polyurethane polyol resin.

4. Writing and/or marking fluid according to one of the preceding claims, wherein one or several of formaldehyde separators, phenoxyethanol, parabens and isothiazolinones are used as the preserving agent.

5. Writing and/or marking fluid according to one of the preceding claims, wherein the silicone wetting agent is a polyethylene-modified polysiloxane.

6. Writing and/or marking fluid according to one of the preceding claims, comprising humectants, in particular glycerine and/or glycols or polyalcohols.

7. Writing and/or marking fluid according to claim 6, wherein up to 15 weight % humectants are added.

8. Writing and/or marking fluid according to one of the preceding claims, comprising further additives selected from anti-blocking additives, in particular a colloidal dispersion of silicon dioxide particles, and/or polyether-modified polydimethylsiloxanes.

9. Writing and/or marking fluid according to claim 8, wherein the further additives are added in a total quantity of up to 10 weight %.

10. Writing and/or marking fluid according to one of the preceding claims, comprising blue dyes or pigments in a concentration of maximum 0.2 weight %.

11. Writing and/or marking fluid according to one of claims 1 to 9, comprising dyes or pigments in a concentration of maximum 3.0 weight %.

12. Writing instrument having a writing tip (3) made of a capillary material and a magazine (2) containing a writing and/or marking fluid according to one of the preceding claims and made of a capillary material.

## Revendications

1. Liquide blanchâtre d'écriture et/ou de marquage, notamment liquide blanc d'écriture et/ou de marquage pour des appareils d'écriture, notamment pour des appareils d'écriture à capillarité ou des applications en cosmétique, qui a une viscosité de moins de 30 mPa s (Brookfield, 20°C, cône-plaque CPE-40), contenant de 45 à 70% en poids d'une dispersion aqueuse de dioxyde de titane, de 5 à 15% en poids d'une dispersion anionique d'une résine durcie, de 1,0 à 3,0% en poids d'un agent de réticulation au silicone et d'un agent de conservation, la teneur en matière solide de la dispersion de dioxyde de titane allant de 70 à 75% en poids.

2. Liquide d'écriture et/ou de marquage suivant la revendication 1, qui contient de 50 à 65% en poids, de préférence de 55 à 60% en poids d'une dispersion aqueuse de dioxyde de titane.

3. Liquide d'écriture et/ou de marquage suivant la revendication 1 ou 2, dans lequel la résine dure est une résine de polyuréthane polyol.

4. Liquide d'écriture et/ou de marquage suivant l'une des revendications précédentes, dans lequel il est utilisé, comme agent de conservation, l'un ou plusieurs d'un précurseur de formaldéhyde, du phénoxyéthanol, du parabène et de l'isothiazolinone.

5. Liquide d'écriture et/ou de marquage suivant l'une des revendications précédentes, dans lequel l'agent de réticulation au silicone est un polysiloxane modifié par du polyéthylène.

6. Liquide d'écriture et/ou de marquage suivant l'une des revendications précédentes, comprenant un agent de maintien de l'humidité, notamment de la glycérine et/ou des glycols ou des polyalcools.

7. Liquide d'écriture et/ou de marquage suivant la revendication 6, dans lequel on ajoute jusqu'à 15% en poids d'agent de maintien de l'humidité.

8. Liquide d'écriture et/ou de marquage suivant l'une des revendications précédentes, comprenant d'autres additifs choisis parmi des additifs antiadhésifs, notamment une dispersion colloïdale de particules de dioxyde de silicium et/ou des polyméthylsiloxanes modifiés au polyétheroxyde.

9. Liquide d'écriture et/ou de marquage suivant la revendication 8, dans lequel les autres additifs sont ajoutés en une quantité totale allant jusqu'à 10% en poids.

10. Liquide d'écriture et/ou de marquage suivant l'une des revendications précédentes, comprenant des colorants ou des pigments bleus en une concentration de 0,2% en poids au maximum.

11. Liquide d'écriture et/ou de marquage suivant l'une des revendications 1 à 9, comprenant des colorants ou des pigments en une concentration de 3,0% en poids au maximum.

12. Instrument d'écriture ayant une pointe (3) d'écriture en un matériau capillaire et un réservoir (2) en un matériau capillaire, contenant un liquide d'écriture et/ou de marquage suivant l'une des revendications précédentes.
